# EUROPÄISCHE PATENTSCHRIFT

(11) **EP 1 110 939 B1**
(45) Veröffentlichungstag und Bekanntmachung des Hinweises auf die Patenterteilung: **10.09.2003**
(21) Anmeldenummer: 00126537.0
(22) Anmeldetag: 11.12.2000
(51) Int. Cl.: C07C 45/00, C07C 49/80

(54) **Verfahren zur Herstellung von Poly(fluoralkyl)-acetophenonen**
Process for the preparation of poly(fluoroalkyl)-acetophenones
Procédé pour la préparation de poly(fluoroalkyl)-acétophénones

(30) Priorität: 22.12.1999 DE 19962011; 27.01.2000 DE 10003321
(43) Veröffentlichungstag der Anmeldung: 27.06.2001
(73) Patentinhaber: BAYER AG, 51368 Leverkusen (DE)
(72) Erfinder: Kühnle, Wulf, Dr., 51061 Köln (DE); Höpfner, Thomas, Dr., 51103 Köln (DE)

(56) Entgegenhaltungen:
- EP-A- 0 949 243
- DE-A- 19 719 054
- "METHOD FOR PREPARATION OF ACETOPHENONE DERIVATIVES" RESEARCH DISCLOSURE, KENNETH MASON PUBLICATIONS, HAMPSHIRE, GB, Nr. 402, 1. Oktober 1997 (1997-10-01), Seite 706 XP000726840 ISSN: 0374-4353

## Beschreibung

Die vorliegende Erfindung betrifft ein verbessertes Verfahren zur Herstellung von Acetophenonen, die am aromatischen Kern mit 2 oder mehr Fluoralkylgruppen substituiert sind. Derartige Verbindungen sind wertvolle Zwischenprodukte zur Herstellung von Wirkstoffen zur Behandlung von Entzündungen, Migräne, Erbrechen und Schmerzen. Insbesondere betrifft die vorliegende Erfindung ein Verfahren zur Herstellung von Bis-3,5-(trifluormethyl)-acetophenon.

Es ist bekannt, dass man Bis-3,5-(trifluormethyl)-acetophenon aus Bis-3,5-(trifluormethyl)-benzoesäurechlorid durch Umsetzung mit organischen Kupferverbindungen herstellen kann (Tetrahedron Letters No. 53, 4647-50 (1970)). Nachteilig bei diesem Verfahren ist die notwendige Herstellung und Verwendung von Lithiumdialkylkupfer-Verbindungen bei -78°C und unter absolutem Wasserausschluss. Derartige Methoden können im Labor, nicht jedoch in technischen Größenordnungen angewendet werden.

Als Verfahren zur Herstellung von Bis-3,5-(trifluormethyl)-acetophenon ist bekannt, dass man aus Fluoralkylanilin und Natriumnitrit in Gegenwart von Schwefelsäure die entsprechende Diazoniumsalzmischung herstellt, dann bei -5 bis ±0°C zu einer Vorlage hinzufügt, die Wasser, Acetaldoxim, ein Kupfer(II)-salz, eventuell ein Reduktionsmittel (Natriumthiosulfat) und in jedem Fall eine große Menge Natriumacetat-Puffer enthält. Zur Aufarbeitung versetzt man mit Salzsäure, erhitzt zum Rückfluss, führt eine Wasserdampfdestillation oder Phasentrennung durch und destilliert im Vakuum. Man erhält so Bis-3,5-(trifluormethyl)-acetophenon in einer Ausbeute von 51 % der Theorie (EP-A1-0 949 243, Beispiel 6-3).

Nachteilig bei diesem Verfahren ist der Einsatz einer großen Menge Hilfsstoffe, z.B. Puffersalze, die die Aufarbeitung erschweren, zu einer erheblichen Salzfracht der Abwässer führen und nach ihrer Abtrennung hohe Kosten für eine umweltgerechte Entsorgung verursachen.

Es ist auch ein Verfahren zur Herstellung von Mono-(fluoralkyl)-acetophenonen bekannt, bei dem man das Verfahren der EP-A1-0 949 243 abwandelt, indem man in Gegenwart von Halogenidionen, z.B. in Gegenwart von Salzsäure, und ohne Zusatz von Puffersalzen arbeitet (DE 197 19 054 A1). Dann treten die o.a. Nachteile nicht auf. Die Übertragung dieses Verfahrens auf die Herstellung von Poly-(fluoralkyl)-acetophenonen hat jedoch nicht nahe gelegen, weil die dann als Ausgangsprodukte benötigten Poly-(fluoralkyl)-aniline schwächere Basen als Mono-(fluoroalkyl)-aniline sind und deshalb zur Bildung von Triazenen neigen. Eine Nacharbeitung des Verfahrens gemäß der DE 197 19054 A1 mit Bis-3,5-(trifluormethyl)-anilin als Ausgangsmaterial ergab Bis-3,5-(trifluoromethyl)-acetophenon in einer Ausbeute von nur 33 % (siehe Beispiel 3). Eine Erhöhung der Salzsäuremenge führte nicht zu einer Verbesserung der Ausbeute.

Es besteht deshalb noch ein Bedarf nach einem einfachen und kostengünstigen, auch in technischem Maßstab durchzuführenden Verfahren zur Herstellung von Acetophenonen, die am aromatischen Kern 2- oder mehrfach mit Fluoralkylgruppen substituiert sind.

Es wurde nun ein Verfahren zur Herstellung von Acetophenonen, die am aromatischen Kern 2- oder mehrfach mit Fluoralkylgruppen substituiert sind, aus entsprechenden Fluoralkylanilinen und Acetaldoxim gefunden, bei dem man aus dem Fluoralkylanilin eine entsprechende Diazoniumsalzmischung herstellt und diese mit Acetaldoxim in Gegenwart wenigstens einer Kupfer- und/oder Palladiumverbindung umsetzt, das dadurch gekennzeichnet ist, dass man keine Puffersalze und kein Reduktionsmittel zusetzt, die Umsetzung mit Acetaldoxim bei 5 bis 50°C und in Gegenwart von Halogenidionen und mindestens einer starken Säure, bei der es sich nicht um eine Halogenwasserstoffsäure handelt, durchführt und abschließend auf eine Temperatur im Bereich 70 bis 160°C erwärmt.

In das erfindungsgemäße Verfahren kann man z.B. Fluoralkylaniline der Formel (I) einsetzen 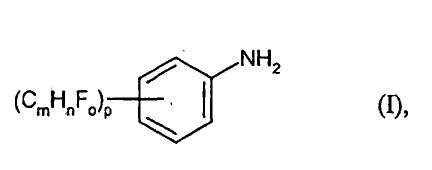 in der
- m: für eine ganze Zahl von 1 bis 4,
- n: für Null oder eine ganze Zahl von 1 bis 2m,
- o: für eine ganze Zahl von 1 bis 2m + 1 und
- p: für eine ganze Zahl von 2 bis 4 stehen,
wobei gilt n + o = 2m + 1.

In der Formel (I) können die vorhandenen CₘHₙFₒ-Reste gleich oder verschieden sein. Vorzugsweise sind sie gleich.

In der Formel (I) stehen
- m: vorzugsweise für 1 oder 2, besonders bevorzugt für 1,
- n: vorzugsweise für Null,
- o: vorzugsweise für 2m + 1 und
- p: vorzugsweise für 2.

Wenn in der Formel (I) p für 2 steht, sind vorzugsweise beide CₘHₙFₒ-Gruppen meta-ständig zur NH₂-Gruppe angeordnet. Wenn in der Formel (I) p für 3 oder 4 steht, sind vorzugsweise zwei der vorhandenen CₘHₙFₒ-Gruppen meta-ständig zur NH₂-Gruppe angeordnet.

Besonders bevorzugt wird als Verbindung der Formel (I) Bis-3,5-(trifluormethyl)-anilin eingesetzt.

Es ist ein wesentliches Merkmal der vorliegenden Erfindung, dass die Umsetzung mit Acetaldoxim in Gegenwart von Halogenidionen und mindestens einer starken Säure, bei der es sich nicht um eine Halogenwasserstoffsäure handelt, erfolgt. Als solche starken Säuren kommen beispielsweise Schwefelsäure, Perchlorsäure, Alkyl- und Arylsulfonsäuren und starke Carbonsäuren infrage. Alkylsulfonsäuren können dabei beispielsweise 1 bis 6 C-Atome enthalten und gegebenenfalls mit Halogenatomen substituiert sein. Arylsulfonsäuren können beispielsweise 6 bis 10 C-Atome enthalten und gegebenenfalls mit Halogenatomen substituiert sein. Bei den starken Carbonsäuren kann es sich beispielsweise um mit Halogenatomen substituierte 2 bis 6 C-Atome enthaltende Alkancarbonsäuren handeln. Einzelbeispiele für Sulfon- und starke Carbonsäuren sind Methansulfonsäure, Trifluormethansulfonsäure, Trichloressigsäure und Trifluoressigsäure. Vorzugsweise verwendet man Schwefelsäure oder Gemische von Schwefelsäure urid einer oder mehrerer anderen von Halogenwasserstoffsäuren verschiedenen starken Säuren. Besonders bevorzugt verwendet man Schwefelsäure.

Man kann beispielsweise so verfahren, dass man bereits die Umsetzung des Fluoralkylanilins mit Natriumnitrit in Gegenwart einer Halogenwasserstoffsäure und mindestens einer starken Säure, bei der es sich nicht um eine Halogenwasserstoffsäure handelt, durchführt. Die Säuren gelangen vorzugsweise in Form konzentrierter wässriger Lösungen zum Einsatz, beispielsweise Salzsäure mit einer Konzentration im Bereich 25 bis 40 Gew.-%, Bromwasserstoffsäure mit einer Konzentration im Bereich 25 bis 70 Gew.-% und Schwefelsäure mit einer Konzentration im Bereich 35 bis 100 Gew.-%. Statt Salzsäure oder Bromwasserstoffsäure kann auch Chlorwasserstoff-Gas oder Bromwasserstoff-Gas in das Reaktionsgemisch eingeleitet werden.

Vorzugsweise setzt man ein Gemisch aus Halogenwasserstoffsäure, insbesondere Salzsäure, und Schwefelsäure ein. Das Mol-Verhältnis solcher Halogenwasserstoff-/Schwefelsäure-Gemische kann beispielsweise im Bereich 1:2 bis 4:1 liegen.

Bezogen auf 1 mol Fluoralkylanilin kann man z.B. 5 bis 9 Äquivalente Protonen in Form von Halogenwasserstoffsäure und mindestens einer starken Säure, bei der es sich nicht um eine Halogenwasserstoffsäure handelt, einsetzen. Vorzugsweise liegt diese Menge bei 5,5 bis 6 Äquivalenten.

Der Wassergehalt des Reaktionsgemisches zur Herstellung der Diazoniumsalzmischung kann beispielsweise 45 bis 80 Gew.-% betragen.

Man kann zur Herstellung der Diazoniumsalzmischung so verfahren, dass man die Säuren und Wasser vorlegt, das Fluoralkylanilin zufügt, anschließend eine wässrige Natriumnitrit-Lösung bei z.B. -20 bis 0°C langsam zufügt und das Gemisch ausreagieren lässt.

Es ist ein weiteres wesentliches Merkmal der vorliegenden Erfindung, dass man der fertigen Diazoniumsalzmischung keine Puffersalze, z.B. kein Natriumacetat, zusetzt.

Für die Umsetzung mit Acetaldoxim kann man, bezogen auf 1 mol eingesetztes Fluoralkylanilin z.B. 1 bis 4 mol Acetaldoxim einsetzen. Vorzugsweise beträgt diese Menge 1,2 bis 3,2 mol.

Als Kupferverbindungen kommen beispielsweise Salze und Komplexverbindungen des Kupfers in Frage, in denen Kupfer in den Oxidationsstufen +1 oder +2 vorliegt.

Beispiele für Kupfersalze sind Kupferhalogenide, Kupfersulfate, Kupfernitrate, Kupfertetrafluoroborate und Kupfersalze organischer Säuren wie Alkyl- und Arylcarbonsäuren. Beispiele für Komplexverbindungen des Kupfers sind solche mit Hydroxylamin- oder Acetaldoxim-Liganden. Im einzelnen seien genannt: Kupfer(II)-sulfat, Kupfer(II)-sulfat-pentahydrat, Kupfer(I)-chlorid, Kupfer(II)-chlorid, Kupfer-(II)-chlorid-dihydrat, Kupfer(I)-bromid, Kupfer(II)-bromid, Kupfer(II)-acetat, Kupfer(II)-acetat-hydrat, Kupfer(II)-fluorid, Kupfer(II)-fluorid-trihydrat, Kupfer(II)-formiat-hydrat, Kupfer(II)-hydroxid, Kupfer(II)-hydroxid-carbonat, Kupfer(II)-nitrat, Kupfer(II)-nitrat-hydrat, Kupfer(II)-nitrat-hemipentahydrat, Kupfer(II)-tetrafluoroborat und Cu(O-N=CH-CH₃)ₙ mit n = 1 oder 2.

Als Palladiumverbindungen kommen beispielsweise Salze und Komplexverbindungen des Palladiums infrage, in denen Palladium in der Oxidationsstufe +2 vorliegt. Beispiele für Palladiumsalze sind Palladiumhalogenide, Palladiumsulfat, Palladiumnitrat und Palladiumsalze organischer Säuren wie Alkyl- und Arylcarbonsäuren. Beispiele für Komplexverbindungen des Palladiums sind solche mit Amin-, Ethylendiamin-, Tetramethylendiamin-, Acetylacetonat- oder Phosphin-Liganden. Im einzelnen seien genannt: Palladium(II)-acetat, -propionat, -chlorid, -nitrat, -sulfat und -trifluoracetat.

Man kann auch Gemische von Kupferverbindungen, Gemische von Palladiumverbindungen oder Gemische von Kupfer- und Palladiumverbindungen einsetzen. Vorzugsweise werden Kupferverbindungen eingesetzt, besonders bevorzugt Kupfer(II)-halogenide oder Kupfer(II)-sulfat, die gegebenenfalls Kristallwasser enthalten können.

Bezogen auf 1 mol eingesetztes Fluoralkylanilin kann man z.B. 0,01 bis 0,2 mol Kupfer- und/oder Palladiumverbindungen einsetzen. Vorzugsweise liegt diese Menge bei 0,04 bis 0,12 mol.

Es ist ein weiteres wesentliches Merkmal der vorliegenden Erfindung, dass man vor und/oder während der Umsetzung der Diazoniumsalzmischung mit dem Acetaldoxim keine Puffersalze (wie Natriumacetat) und keine Reduktionsmittel (wie Natriumthiosulfat) hinzufügt.

Die Umsetzung mit dem Acetaldoxim wird vorzugsweise bei 5 bis 40°C, insbesondere bei 10 bis 30°C durchgeführt.

Die erfindungsgemäß erforderliche Gegenwart von Halogenidionen während der Umsetzung mit Acetaldoxim kann im einfachsten Fall dadurch erreicht werden, dass man für die Herstellung der Diazoniumsalzmischung als Säure eine Halogenwasserstoffsäure (mit-)verwendet, beispielsweise 25 bis 40 gew.-%ige wässrige Salzsäure oder 25 bis 70 gew.-%ige wässrige Bromwasserstoffsäure. Wenn man bei der Herstellung der Diazoniumsalzmischung nur andere Säuren als Halogenwasserstoffsäuren eingesetzt hat, muss man Halogenidionen zusetzen, beispielsweise in Form von Alkali- oder Erdalkalimetallhalogeniden, etwa in Form von Natriumchlorid oder Natriumbromid.

Bezogen auf 1 mol eingesetzte Kupfer- und/oder Palladiumverbindungen kann das Reaktionsgemisch der Umsetzung mit dem Acetaldoxim beispielsweise 1 bis 50 mol Halogenidionen enthalten. Vorzugsweise liegt diese Menge bei 1,5 bis 35 mol. Wenn die Diazoniumsalzmischung nicht genügend Halogenidionen mitbringt, sind diese gesondert hinzuzufügen.

Die Umsetzung mit dem Acetaldoxim führt man vorzugsweise so durch, dass man die Kupfer- und/oder Palladiumverbindungen, Wasser und Acetaldoxim vorlegt und die wässrige Diazoniumsalzmischung langsam zudosiert. Es ist vorteilhaft, während des Zudosierens der Diazoniumsalzmischung für eine intensive Durchmischung des Reaktionsgemisches zu sorgen, beispielsweise durch kräftigen Rühren oder mittels eines Vibrationsmischers oder eines Dispergiergerätes, z.B. der Art Ultra-Turrax ®.

Es ist weiterhin vorteilhaft, bei der Bereitung der miteinander umzusetzenden Komponenten und während der Reaktion die Gegenwart von Sauerstoff weitgehend auszuschließen. Man kann hierzu beispielsweise in einer Inertgasatmosphäre arbeiten, Inertgas in die zu handhabenden Komponenten und/oder Reaktionsgemische einleiten oder ein leichtes Vakuum zur Entgasung anlegen.

Nach Beendigung der Zugabe der Diazoniumsalzmischung und des Acetaldoxims kann man das Reaktionsgemisch gegebenenfalls noch einige Zeit, beispielsweise 15 Minuten bis 1 Stunde und z.B. bei Temperaturen im Bereich 20 bis 50°C nachreagieren lassen. Ein Versetzen mit (weiterer) Salzsäure vor der Aufarbeitung ist nicht erforderlich.

Zur Aufarbeitung wird das ausreagierte Reaktionsgemisch erfindungsgemäß bei erhöhter Temperatur getempert; wobei auf 70 bis 160°C - gegebenenfalls unter Druck -, bevorzugt auf 70 bis 110°C erwärmt und der Ansatz z.B. 15 Minuten bis 12 Stunden, bevorzugt 1 bis 4 Stunden in diesem Temperaturbereich gehalten wird. Zur Gewinnung des Rohproduktes kann während des Tempems oder anschließend das ausreagierte Reaktionsgemisch mittels Wasserdampfdestillation oder Azeotropdestillation aufgearbeitet werden. Alternativ kann das ausreagierte Reaktionsgemisch nach dem Tempern auf z.B. 0-70°C abgekühlt werden und das Rohprodukt durch Phasentrennung, Extraktion oder - bei festen Produkten - Filtration gewonnen werden.

Die nach der Aufarbeitung, z.B. durch Wasserdampfdestillation, Phasentrennung, Extraktion oder Filtration, vorliegenden rohen Acetophenone, die am Kern 2- oder mehrfach mit Fluoralkylgruppen substituiert sind, kann man gegebenenfalls weiter reinigen, z.B. durch fraktionierende Destillation über eine Kolonne.

Man kann auf die erfindungsgemäße Weise die gewünschten Produkte in Reinheiten von im allgemeinen über 98 % und in Ausbeuten von im allgemeinen 50 bis 58 % der Theorie erhalten.

Wenn man ein Fluoralkylanilin der Formel (I) eingesetzt hat, dann erhält man das entsprechende Acetophenon der Formel (II) 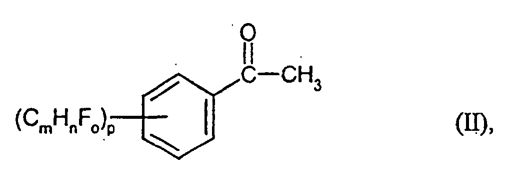 in der die verwendeten Symbole die bei Formel (I) angegebene Bedeutung haben.

Obwohl das erfindungsgemäße Verfahren im Gegensatz zum Stand der Technik ohne Zusatz von Puffersalzen und Reduktionsmittel auskommt und mit geringeren Mengen starker anorganischer Säuren und Kupferverbindungen durchgeführt werden kann, ist bei ihm die Ausbeute an Zielprodukten im allgemeinen um mindestens 20 % (relativ) erhöht. Das ist besonders überraschend und macht das erfindungsgemäße Verfahren wesentlich effizienter als die bekannten Verfahren. Die Abwassermengen und deren Salzgehalte sind wesentlich geringer, die Raumausbeute ist stark erhöht. Die erfindungsgemäß gegenüber dem Stand der Technik erhöhte Reaktionstemperatur ließ ein Ansteigen von Zersetzungsreaktionen erwarten, was überraschenderweise nicht eintritt.

### Beispiele

### Beispiel 1

274 g Wasser, 171 g 30 gew.-%ige wässrige Salzsäure und 69 g 98 gew.-%ige wässrige Schwefelsäure wurden vorgelegt und auf -5°C gekühlt. Dann dosierte man bei dieser Temperatur 115 g Bis-3,5-(trifluormethyl)-anilin mit einem Gehalt von 99 % zu. Nunmehr wurden 35,6 g Natriumnitrit in 110 g Wasser gelöst und diese Lösung im Verlauf einer Stunde bei 0°C eindosiert und 1 Stunde nachgerührt. Aus 5,8 g Kupfer(II)-sulfat-pentahydrat, 172 g Wasser und 44,4 g Acetaldoxim wurde eine Vorlage bereitet und auf 20°C erwärmt. In diese Vorlage wurde die zuvor hergestellte Diazoniumsalzlösung unter intensivem Rühren im Verlauf von 2 Stunden eindosiert und danach 30 Minuten bei 30°C nachreagieren gelassen. Danach erwärmte man im Verlauf von 2 Stunden auf 100°C und rührte 3 Stunden bei dieser Temperatur nach. Nach dem Abkühlen auf 50°C wurden die Phasen getrennt. Die organische Phase über eine Kolonne destilliert und so 71,1 g Bis-3,5-(trifluormethyl)-acetophenon erhalten (gemäß GC 99 %ig). Das entspricht einer Ausbeute von 55 % d. Th.

### Beispiel 2

Eine Diazoniumsalzlösung wurde wie in Beispiel 1 beschrieben hergestellt.

Aus 8,7 g Kupfer(II)-sulfat-pentahydrat, 172 g Wasser, 100 g Toluol und 44,4 g Acetaldoxim wurde eine Vorlage hergestellt, auf 10°C abgekühlt und danach im Verlauf von 1,5 Stunden die Diazoniumsalzlösung zugetropft. Anschließend erhitzte man auf 100°C und rührte 3 Stunden bei dieser Temperatur nach. Nach dem Abkühlen auf 50°C trennte man die Phasen. Die organische Phase wurde über eine Kolonne destilliert. So wurden 64,6 g Bis-3,5-(trifluormethyl)-acetophenon (gemäß GC 99 % rein). Das entspricht einer Ausbeute von 50 % der Theorie.

### Beispiel 3 (zum Vergleich)

### (Arbeitsweise entsprechend DE 197 19 054 A1, d.h. nur mit Salzsäure)

254 g Wasser und 133 g wässrige 30 gew.-%ige Salzsäure wurden vorgelegt und auf -5°C abgekühlt. Dann wurden bei dieser Temperatur 57,3 g Bis-3,5-(trifluormethyl)-anilin mit einem Gehalt von 99 % zugetropft. Danach wurden 18,3 g Natriumnitrit in 54,8 g Wasser gelöst und im Verlauf einer Stunde bei 0°C zu dem Bis-3,5-(trifluormethyl)-anilin-enthaltenden Gemisch zudosiert und eine Stunde nachgerührt. Aus 2,9 g Kupfer(II)-sulfat-pentahydrat, 86,2 g Wasser und 22,2 g Acetaldoxim wurde eine Vorlage bereitet und bei 25°C intensiv gerührt. Dazu wurde im Verlauf von 2 Stunden die zuvor bereitete Diazoniumsalzlösung eindosiert und 30 Minuten bei 30°C nachreagieren gelassen. Im Verlauf von 2 Stunden erwärmte man das Gemisch auf 100°C und rührte 3 Stunden bei dieser Temperatur nach. Nach dem Abkühlen auf 50°C wurden die Phasen getrennt. Die organische Phase wurde über eine Kolonne destilliert und so 21,3 g Bis-3,5-(trifluormethyl)-acetophenon erhalten (Gehalt nach GC 99 %). Das entspricht einer Ausbeute von 33 % d. Th.

## Patentansprüche

1. Verfahren zur Herstellung von Acetophenonen, die am aromatischen Kern 2- oder mehrfach mit Fluoralkylgruppen substituiert sind, aus den entsprechenden Fluoralkylanilinen und Acetaldoxim, bei dem man aus dem Fluoralkylanilin eine entsprechende Diazoniumsalzmischung herstellt und diese mit Acetaldoxim in Gegenwart wenigstens einer Kupfer- und/oder Palladiumverbindung umsetzt, **dadurch gekennzeichnet, dass** man keine Puffersalze und kein Reduktionsmittel zusetzt, die Umsetzung mit Acetaldoxim bei 5 bis 50°C und in Gegenwart von Halogenidionen und mindestens einer starken Säure, bei der es sich nicht um eine Halogenwasserstoffsäure handelt, durchführt und abschließend auf eine Temperatur im Bereich 70 bis 160°C erwärmt.

2. Verfahren nach Anspruch 1, **dadurch gekennzeichnet, dass** man Fluoralkylaniline der Formel (I) 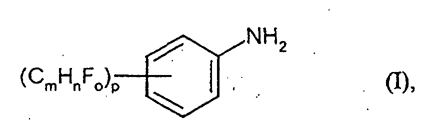 in der
m für eine ganze Zahl von 1 bis 4,
n für Null oder eine ganze Zahl von 1 bis 2m,
o für eine ganze Zahl von 1 bis 2m + 1 und
p für eine ganze Zahl von 2 bis 4 stehen,
wobei gilt n + o = 2m + 1
einsetzt und Acetophenone der Formel (II) 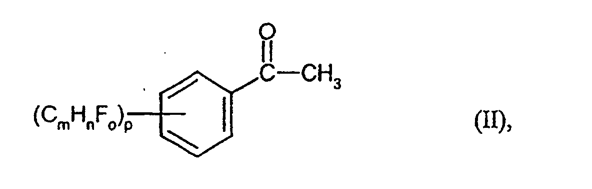 in der die verwendeten Symbole die bei Formel (I) angegebene Bedeutung haben,
herstellt.

3. Verfahren nach Anspruch 2, **dadurch gekennzeichnet, dass** in den Formeln (I) und (II)
m für 1 oder 2,
n für Null,
o für 2m + 1 und
p für 2 stehen.

4. Verfahren nach Ansprüchen 1 bis 3, **dadurch gekennzeichnet, dass** man ein Gemisch aus Salzsäure und Schwefelsäure einsetzt.

5. Verfahren nach Ansprüchen 1 bis 4, **dadurch gekennzeichnet, dass** man pro mol eingesetztes Fluoralkylanilin 1 bis 4 mol Acetaldoxim und 0,01 bis 0,2 Kupfer- und/oder Palladiumverbindungen einsetzt.

6. Verfahren nach Ansprüchen 1 bis 5, **dadurch gekennzeichnet, dass** man die Umsetzung mit Acetaldoxim bei 5 bis 40°C durchführt.

7. Verfahren nach Ansprüchen 1 bis 6, **dadurch gekennzeichnet, dass** man bezogen auf 1 mol eingesetzte Kupfer- und/oder Palladiumverbindungen 1 bis 50 mol Halogenidionen einsetzt.

8. Verfahren nach Ansprüchen 1 bis 7, **dadurch gekennzeichnet, dass** man bei der Bereitung der miteinander umzusetzenden Komponenten während der Reaktion die Gegenwart von Sauerstoff weitgehend ausschließt.

9. Verfahren nach Ansprüchen 1 bis 8, **dadurch gekennzeichnet, dass** man die abschließende Erwärmung auf 70 bis 160°C für 15 Minuten bis 12 Stunden durchführt.

10. Verfahren nach Ansprüchen 1 bis 9, **dadurch gekennzeichnet, dass** man Kupferverbindungen einsetzt.

## Claims

1. Process for the preparation of acetophenones which are di- or polysubstituted by fluoroalkyl groups on the aromatic ring from the corresponding fluoroalkylanilines and acetaldoxime, in which a corresponding diazonium salt mixture is prepared from the fluoroalkylaniline and is reacted with acetaldoxime in the presence of at least one copper and/or palladium compound, **characterized in that** no buffer salts and no reducing agent is/are added, the reaction with acetaldoxime is carried out at 5 to 50°C and in the presence of halide ions and at least one strong acid which is not a hydrohalic acid, and finally the mixture is heated to a temperature in the range 70 to 160°C.

2. Process according to Claim 1, **characterized in that** fluoroalkylanilines of the formula (I) 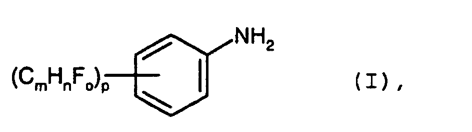 in which
m is an integer from 1 to 4,
n is zero or an integer from 1 to 2m,
o is an integer from 1 to 2m + 1 and
p is an integer from 2 to 4,
where n + o = 2m + 1
are used, and acetophenones of the formula (II) 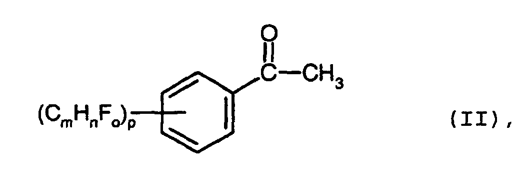 in which the symbols used have the meanings given for formula (I),
are prepared.

3. Process according to Claim 2, **characterized in that** in the formulae (I) and (II)
m is 1 or 2,
n is zero,
o is 2m + 1 and
p is 2.

4. Process according to Claims 1 to 3, **characterized in that** a mixture of hydrochloric acid and sulphuric acid is used.

5. Process according to Claims 1 to 4, **characterized in that**, per mole of fluoroalkylaniline used, 1 to 4 mol of acetaldoxime and 0.01 to 0.2 mol of copper and/or palladium compounds are used.

6. Process according to Claims 1 to 5, **characterized in that** the reaction with acetaldoxime is carried out at 5 to 40°C.

7. Process according to Claims 1 to 6, **characterized in that**, based on 1 mol of copper and/or palladium compounds used, 1 to 50 mol of halide ions are used.

8. Process according to Claims 1 to 7, **characterized in that** in the preparation of the components to be reacted with one another, the presence of oxygen is largely excluded during the reaction.

9. Process according to claims 1 to 8, **characterized in that** the final heating to 70 to 160°C is carried out for 15 minutes to 12 hours.

10. Process according to claims 1 to 9, **characterized in that** copper compounds are used.

## Revendications

1. Procédé pour la préparation d'acétophénones, qui sont substituées au noyau aromatique, 2 ou plusieurs fois, par des groupes fluoralkyle, à partir des fluoralkylanilines et acétaldoximes correspondants, dans lequel on produit à partir de la fluoralkylaniline un mélange correspondant de sels de diazonium et on fait réagir celui-ci avec l'acétaldoxime en présence, au moins, d'un composé de cuivre et/ou de palladium, **caractérisé en ce que** on n'additionne pas de sels tampons ni de réducteurs, qu'on effectue la réaction avec l'acétaldoxime entre 5 et 50°C et en présence d'ions halogénures et d'au moins un acide fort, qui ne peut pas être un hydracide halogéné , et on réchauffe finalement à une température dans la gamme de 70 à 160°C.

2. Procédé selon la revendication 1, **caractérisé en ce qu'**on utilise de la fluoralkylaniline de formule (I) 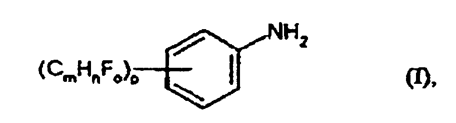 dans laquelle
m représente un nombre entier de 1 à 4,
n représente zéro ou un nombre entier de 1 à 2m,
o représente un nombre entier de 1 à 2m + 1 et
p représente un nombre entier de 2 à 4,
où n + o = 2m + 1
et qu'on produit de l'acétophénone de formule (II) 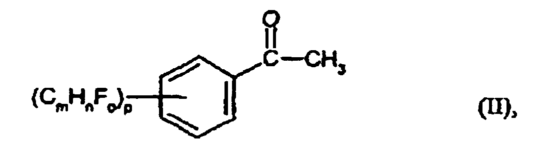 dans laquelle les symboles utilisés ont la signification donnée pour la formule (I).

3. Procédé selon la revendication 2, **caractérisé en ce que** dans les formules (I) et (II)
m représente 1 ou 2,
n représente zéro,
o représente 2m + 1 et
p représente 2.

4. Procédé selon les revendications 1 à 3, **caractérisé en ce qu'**on utilise un mélange d'acide chlorhydrique et d'acide sulfurique.

5. Procédé selon les revendications 1 à 4, **caractérisé en ce que**, par mole de fluoralkylaniline utilisée, on utilise de 1 à 4 moles d'acétaldoxime et de 0,01 à 0,2 mole de composés de cuivre et/ou de palladium.

6. Procédé selon les revendications 1 à 5, **caractérisé en ce qu'**on effectue la réaction avec l'acétaldoxime entre 5 et 40°C.

7. Procédé selon les revendications 1 à 6, **caractérisé en ce que**, par rapport à 1 mole de composés de cuivre et/ou de palladium utilisés, on utilise de 1 à 50 moles d'ions halogénures.

8. Procédé selon les revendications 1 à 7, **caractérisé en ce que**, dans la préparation des composants à faire réagir ensemble et pendant la réaction, on exclut dans une large mesure la présence d'oxygène.

9. Procédé selon les revendications 1 à 8, **caractérisé en ce qu'**on effectue le réchauffement final entre 70 et 160°C pendant une période de 15 minutes à 12 heures.

10. Procédé selon les revendications 1 à 9, **caractérisé en ce qu'**on utilise des composés de cuivre.
